# EUROPÄISCHE PATENTSCHRIFT

(11) **EP 2 380 526 B1**
(45) Veröffentlichungstag und Bekanntmachung des Hinweises auf die Patenterteilung: **02.01.2019**
(21) Anmeldenummer: 11160805.5
(22) Anmeldetag: 01.04.2011
(51) Int. Cl.: A61L 31/06, A61F 2/90, A61F 2/07, A61L 31/00

(54) **Implantat und Verfahren zur Herstellung desselben**
Implant and method for producing the same
Implant et son procédé de fabrication

(30) Priorität: 23.04.2010 US 327113 P
(43) Veröffentlichungstag der Anmeldung: 26.10.2011
(60) Teilanmeldung: 18207291.8
(73) Patentinhaber: Biotronik AG, 8180 Bülach (CH)
(72) Erfinder: Wintsch, Daniel, 8154 Oberglatt (CH); Loher, Stefan, 8006, Zürich (CH); Quint, Bodo, 8154, Oberglatt (CH); Bachmann, Patrice, 8400, Winterthur (CH); Lang, Hans, 8107, Buchs (CH); Pantic, Dragica, 8051, Zürich (CH); Schwitzer, Alwin, 8180, Bülach (CH)
(74) Vertreter: Galander, Marcus

(56) Entgegenhaltungen:
- EP-A1- 1 493 404
- EP-A2- 2 088 962
- WO-A1-93/08767
- WO-A1-97/24081
- WO-A1-03/087443
- WO-A1-2006/065665
- US-B1- 6 325 823

## Beschreibung

Die vorliegende Erfindung betrifft ein Implantat, insbesondere eine intraluminale Endoprothese, mit einer hohlzylinderischen Grundstruktur sowie ein Verfahren zur Herstellung eines derartigen Implantats.

Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents (Gefäß-Stent, Gallengang Stent, vascular Stent, Mitral-Stent), Endoprothesen zum Verschließen von persistierenden Foramen ovale (PFO), Pulmonalklappenstent (pulmonary valve stent), Endoprothesen zum Verschließen eines ASD (Vorhofscheidewanddefekt, atrial septal defect), Prothesen im Bereich des Hart- und Weichgewebes sowie Ankerelemente für Elektroden, insbesondere von Schrittmachern oder Defibrillatoren zu verstehen.

Heutzutage werden als Implantate besonders häufig Stents eingesetzt, die zur Behandlung von Stenosen (Gefäßverengungen) dienen. Sie weisen einen Körper in der Form einer ggf. durchbrochenen rohrförmigen oder hohlzylinderförmigen Grundstruktur auf, die an beiden Längsenden offen ist. Die Grundstruktur des Stents kann sich aus einzelnen Maschen zusammensetzen, welche aus zick-zack- oder mäander-förmigen Stegen gebildet werden. Die rohrförmige Grundstruktur einer derartigen Endoprothese wird in das zu behandelnde Gefäß eingesetzt und dient dazu, das Gefäß zu stützen. Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents oder anderen Implantaten kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die beispielsweise eine Entzündung (Inflammation) des behandelten Gefäßes oder eine nekrotische Gefäßveränderung begünstigen und die zu einem allmählichen Zuwachsen des Stents durch Bildung von Plaques führen können.

Als Stentgrafts werden Stents bezeichnet, welche auf oder in ihrer häufig gitterförmigen Grundstruktur noch ein Vlies oder eine andere flächige Bedeckung, wie beispielsweise eine Folie, enthalten. Hierbei wird unter einem Vlies ein textiles Flächengebildet verstanden, welches durch einzelne Fasern gebildet wird. Mit dem Begriff Vlies ist bei der vorliegenden Erfindung auch der Fall umfasst, bei dem das textile Flächengebilde nur aus einer einzigen "Endlos"-Faser besteht. Ein solcher Stentgraft wird beispielsweise eingesetzt, um Schwachpunkte in Arterien zu stützen, beispielsweise im Bereich eines Aneurysmas oder einer Ruptur der Gefäßwand (Bail-Out-Device), insbesondere als Notfall-Stent.

Bekannte Stents, welche als Stentgraft mit einer PTFE-Folie oder einer ePTFE-Folie versehen sind, zeigen auf Grund der Folienstruktur ein schlechtes Einwachsverhalten im vaskulären System. In einer aus dem Stand der Technik bekannten Variante eines Stentgrafts ist beispielsweise eine ePTFE-Folie zwischen zwei Stent-Gitterstrukturen sandwichartig angeordnet. Es zeigt sich, dass ein solcher Stentgraft sehr steif ist, so dass er aufgrund der erhöhten Biegesteifigkeit der Sandwich-Struktur eine erhöhte Restenose-Rate aufweist. Die ePTFE-Folienstruktur zeigt eine schlechtere Endothelialisierung und eine schlechtere transmurale Kommunikation. Ferner ist ein solcher "Sandwich"-Stentgraft schlecht oder überhaupt nicht an der gewünschten Läsion zubringbar, insbesondere bei tortuösen Gefäßanatomien.

In der Druckschrift WO 03/087443 A1 wird eine faserartige Abdeckung für einen Stent beschrieben, die mittels Elektrospinnen auf die Tragstruktur aufgebracht wird. Als Fasermaterialien werden in dieser Druckschrift PET, PLA, PGA oder PCL verwendet. Obwohl die aus diesen Materialien hergestellten Stentgrafts eine vergleichsweise hohe Porosität aufweisen, so dass in kritischen Bereichen der Fluss in den Kapillaren der Wand des behandelten Gefäßes gewährleistet und somit eine gute Endothelialisierung erreichbar ist, wurde bei einem solchen elektrogesponnenen Stentgraft eine erhöhte Komplikationsrate beobachtet.

Aus dem Dokument CA 2 567 954 A1 ist ein Stentgraft bekannt, bei dem mittels Annähen des Graftmaterials im Bereich der Stege und/oder mittels einer Unterlegscheibe an einem Steg eine bessere Anbindung des Graftmaterials an dem jeweiligen Steg bewirkt werden soll. Die Komplikationsrate konnte mit einer derartigen Festlegung des Graftmaterials jedoch nicht verringert werden.

WO 93/08767 beschreibt ein Verfahren und eine Vorrichtung zur endovaskulären Reparatur von Aneurysmen.

EP 2 088 962 beschreibt eine Stentbaugruppe.

Die Aufgabe der vorliegenden Erfindung besteht somit darin, ein Implantat zu schaffen, das bei weiterhin guter Endothelialisierung eine geringere Komplikationsrate aufweist. Ferner soll ein einfaches Verfahren für die Herstellung eines derartigen Implantats angegeben werden.

Die obige Aufgabe wird durch ein Implantat ausgebildet als intraluminale Endoprothese mit einer hohlzylinderförmigen Grundstruktur, wobei in und/oder auf der Grundstruktur ein Vlies angeordnet ist, welches biokompatibles thermoplastisches Polyurethan und/oder ein Polymer aus der Familie der Polymilchsäure oder Poly-caprolactone oder deren Copolymere und/oder ein Polymer aus der Familie der Polyphosphazene enthält, wobei in mindestens einem Abschnitt des Implantats am proximalen und/oder am distalen Ende des Implantats ein Befestigungsmittel derart vorgesehen ist, dass zumindest in einem expandierten Zustand des Implantats ein Abragen von Vliesabschnitten von der Grundstruktur und/oder die Bildung von größer ausgedehnten Bereichen, in denen Fasern oder Faserabschnitte von der Grundstruktur abragen, verringert wird, wobei das Befestigungsmittel durch ein elastisches Element, vorzugsweise einen elastischen Ring, gebildet ist.

Es ist das Verdienst der Erfinder, erkannt zu haben, dass sich ohne weitere, insbesondere großflächig, das heißt auch zwischen den Maschen wirkende Befestigungsmaßnahmen aus dem Vlies sogenannte Flaps (Klappen) beispielsweise an dem Stentgraft bilden, die insbesondere durch das Spannen des Stents beim Expandieren oder Dilatieren erzeugt werden. Diese Flaps oder größer ausgedehnte Bereiche mit abragenden Faser oder Faserabschnitten, welche insbesondere am distalen oder proximalen Ende des Stents entstehen können, werden aufgrund des durch die Flussgeschwindigkeit der Körperflüssigkeit entstehenden Drucks in das behandelte Gefäß hinein gezogen und behindern hierdurch den Durchfluss der Körperflüssigkeit, z.B. des Bluts, durch das behandelte Gefäß. Ein solcher Vorgang erzeugt eine erhöhte Thrombosegefahr.

Aber auch wenn keine Flaps an einem Stentgraft beobachtet werden, können an den Oberflächen häufig in größer ausgedehnten Bereichen lose Faserabschnitte der Endlosfaser oder lose Einzelfasern auftreten, welche ebenfalls den Durchfluss der Körperflüssigkeit beeinflussen können.

Deshalb wird erfindungsgemäß in einem Abschnitt oder mehreren Abschnitten des Implantats, welches oder welche besonders anfällig für eine erhöhte Thrombosen-Gefahr sind, am proximalen und/oder am distalen Ende des Implantats, das Vlies an die Grundstruktur so angebunden bzw. behandelt, dass sich das Vlies, lose Fasern oder Faserabschnitte insbesondere bei der Zuführung, Positionierung und Freisetzung des Implantats sowie während der vorgesehenen Funktionsdauer nicht von der Grundstruktur ablösen können. Hierdurch werden Komplikationen vermieden. Zudem wird die Restenose-Rate durch eine verbesserte Hämodynamik verringert, insbesondere wenn der Abschnitt am proximalen oder distalen Ende des Implantats sitzt.

Eine signifikante Verringerung des Abragens von Vliesabschnitten oder größer ausgedehnten Fasern, Faserabschnitten mit einer Länge in der Regel von Bruchteilen eines Millimeters in dem Abschnitt des Implantats, der behandelt oder mit einem Befestigungsmittel versehen wird, bedeutet, dass vorzugsweise mindestens 90% weniger, besonders bevorzugt keine, Fasern, Faserabschnitte oder Vliesabschnitte von dem Implantat abragen und/oder dass der durchschnittliche Abstand der abragenden Fasern, Faserabschnitte oder Vliesabschnitte ihres am weitesten abragenden Endes oder Bereichs, mit dem diese in das Lumen des behandelten Gefäßes hineinragen, von der Grundstruktur signifikant, vorzugsweise mindestens um 90%, besonders bevorzugt vollständig, verringert ist jeweils gegenüber dem Zustand ohne Behandlung oder Befestigungsmittel.

Die in der CA 2 567 954 A1 beschriebenen Maßnahmen können derartige Flaps durch das Annähen vermeiden, sie können aber größer ausgedehnte lose Faserabschnitte oder lose Fasern aufgrund ihrer Wirkung lediglich im Bereich der Stege der Grundstruktur nicht vermeiden. Weiterhin ist das Verfahren sehr aufwendig und fehlerbehaftet.

Die erfindungsgemäße Lösung hat den Vorteil, dass das Vlies, insbesondere wenn es einen biokompatiblen Thermoplasten, vorzugsweise ein thermoplastisches Elastomer (TPE), besonders bevorzugt ein thermoplastisches Polyurethan, und/oder ein bioabbaubares Polymer, vorzugsweise ein Polymer aus der Familie der Polymilchsäure oder Polycaprolactone oder deren Copolymere (z.B. PCL/TMC), und/oder ein biokompatibles, anorganisches Polymer, vorzugsweise aus der Familie der Polyphosphazene, enthält, eine gewisse Elastizität aufweist, welche das Abreißen des Vlieses insbesondere beim Dilatieren des Stentgrafts verhindert. Gleichzeitig wird das Recoil des Implantats durch die zusätzliche Vlieshülle im Vergleich zur Grundstruktur ohne Vlies nicht negativ beeinflusst.

Gleichzeitig besitzt das Implantat auf Grund der Verwendung von Vlies nach wie vor eine gezielt beeinflussbare Porosität, so dass in kritischen Bereichen eine transmurale Kommunikation ermöglicht wird.

Derartige Implantate können beispielsweise als sogenannter Bail-Out-Stent, Neurostent, Drug-Eluting-Stent, Graft auf Ballon (PEB), PTA (perkutane transluminale Angeoplastie), als Arterienersatz oder Venenersatz, Ankerelemente von Herzschrittmacher- bzw. Defibrillator-Elektroden eingesetzt werden.

In einem bevorzugten Ausführungsbeispiel ist das Befestigungsmittel durch eine proximal und distal im Inneren der Grundstruktur oder außen auf der Grundstruktur angeordnete Torsionsfeder gebildet. Eine derartige Torsionsfeder ist vorzugsweise als Druckfeder ausgebildet, welche bei einem hohlzylinderförmigen Implantat in das Innere des Implantats eingesetzt wird und auf die Innenwand des Implantats drückt. Hierbei weist die Torsionsfeder vorzugsweise Flächenelemente auf, welche die Form eines Hohlzylinderabschnitts aufweisen und welche auch im expandierten Zustand des Implantats die innere oder die äußere Oberfläche des Implantats derart bedecken, dass Fasern, Faserabschnitte oder Vliesabschnitte von der Grundstruktur nicht in das Innere des Implantats abragen können.

Es können auch mehrere solche Torsionsfedern im Inneren der Grundstruktur und/oder außen auf der Grundstruktur angeordnet werden.

Vorzugsweise besteht eine derartige Torsionsfeder aus dem gleichen Material wie die Grundstruktur, besonders bevorzugt wird sie parallel zur Herstellung der Grundstruktur ohne zusätzliche Befestigungsschritte gefertigt. Vorzugsweise besitzt eine derartige Torsionsfeder einen E-Modul von etwa 30 GPa bis etwa 230 GPa.

In einem weiteren Ausführungsbeispiel werden Flaps dadurch vermieden, dass die Grundstruktur in dem mindestens einen Abschnitt des Implantats im Wesentlichen in Umfangsrichtung verlaufende Konnektoren aufweist, welche als Befestigungsmittel dienen. Der Vorteil derartiger zusätzlicher Konnektoren besteht darin, dass das Vlies in dem jeweiligen Abschnitt durch die Grundstruktur und die Konnektoren abgestützt wird, wodurch Flaps vermieden werden. Diese Konnektoren können wie die oben beschriebene Torsionsfeder aus dem gleichen Material wie die Grundstruktur hergestellt sein. Diese Konnektoren liegen in dem mindestens einen Abschnitt zusätzlich zu den ggf. ebenfalls in Umfangsrichtung verlaufenden Elementen (Stege, Struts) der Grundstruktur vor. Solche Konnektoren weisen vorzugsweise eine ihrer Aufgabe angepasste Struktur und/oder Form auf. So können diese Konnektoren beispielsweise eine gegenüber den Struts um mindestens 50% reduzierte Wandstärke haben, vorzugsweise eine um mindestens 70% gegenüber den Struts reduzierte Wandstärke. Diese dünnwandigen Konnektoren sind sehr flexibel, wodurch sich eine Vielzahl von Konnektoren in die Stentstruktur einbauen lassen, ohne dass der Stent beim Öffnen behindert wird. Zudem kann mit solchen Konnektoren aufgrund ihrer geringen Wandstärke bei der Montage des Stentgrafts auf den Ballon ein Profil mit einer geringen Höhe realisiert werden.

In einem weiteren bevorzugten Ausführungsbeispiel ist das Befestigungsmittel durch zusätzliche, insbesondere im Wesentlichen axial verlaufende Stegelemente gebildet, die zusätzlich zu der Grundstruktur in dem betreffenden Abschnitt angeordnet sind. Auf Grund der erhöhten Anzahl der axialen Abstützungen zu der Grundstruktur in dem mindestens einen Abschnitt des Implantats erfolgt eine bessere mechanische Fixierung des Vlieses an der (in dem Abschnitt veränderten) Gefäßwand nach erfolgter Dilatation. Hierdurch wird ein Lösen des Vlieses von der Grundstruktur weniger kritisch und die Gefahr der Thrombosenbildung durch Flaps reduziert. Besonders bevorzugt verringern diese axial verlaufenden Stegelemente eine Maschenlänge in Umfangsrichtung auf den halben Betrag.

Es ist weiterhin bevorzugt, wenn das Befestigungsmittel durch eine mindestens teilweise vorgesehene Beschichtung gebildet wird, welche an der Außenseite des Vlieses angeordnet ist und welche ein Material enthält, das ein Hydrogel und/oder einen hydrogelbasierten Klebstoff bildet. Dies bedeutet, dass die Beschichtung in dem mindestens einen Abschnitt des Implantats, in dem das Befestigungsmittel angeordnet ist, mindestens einen Teil der äußeren Mantelfläche des Vlieses abdeckt. Die Beschichtung kann in dem jeweiligen Abschnitt des Implantats auch die gesamte Mantelfläche des Vlieses abdecken.

Der Vorteil dieses Ausführungsbeispiels besteht darin, dass das Material mit dem Hydrogel durch Wasseraufnahme aus der Körperflüssigkeit quillt, so dass eine attraktive Wechselwirkung zwischen der äußeren Oberfläche des Implantats (d.h. der äußeren Oberfläche des Vlieses) und der Gefäßwand entsteht. Hierdurch wird das Implantat förmlich an die Gefäßwand geklebt. Durch diese Maßnahme kann die Entstehung von Flaps verhindert, da das Vlies an der Gefäßwand festgelegt ist, und die Gefahr einer Thrombosebildung verringert werden. Vorzugsweise enthält das Material ein oder mehrere Verbindungen der Gruppe enthaltend Polyethyleneoxide, Polyvinylpyrrolidone, Polyvinylalkohol, Polyacrylate, Agarose, Methylcellulose, Polyasparaginsäure, Polyhyaluronsäure, Pullulan, Polysaccharide, insbesondere Dextran. Es ist auch denkbar, dass diese Hydrogele eine Verbindung mit einer pharmazeutisch aktiven Substanz wie Heparin bzw. Heparansulfat eingeht oder das Einbetten einer solchen Substanz erleichtert. Hierdurch wird eine zusätzliche Funktion der Beschichtung realisiert.

Unter einer pharmazeutisch aktiven Substanz (oder therapeutisch aktive oder wirksame Substanz) wird ein pflanzlicher, tierischer oder synthetischer Wirkstoff (Medikament) oder ein Hormon verstanden, das in geeigneter Dosierung als Therapeutikum zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Endoprothesenumgebung hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können in besonders bevorzugten Ausführungsbeispielen aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe der Taxole oder Taxane, hier vorzugsweise Paclitaxel oder Sirolimus, bestehen.

Ein Material mit Hydrogel wird beispielsweise aus einer Lösung (z.B. 5 - 20 Gew.% des Polymers in der Lösung) mit einem für die das Hydrogel bildenden Polymere geeigneten Lösungsmittel (z.B. speziell getrocknetem Chloroform, DMF, THF) in Anwesenheit eines Vernetzungsagens (wie z.B. Hexamethylendiisocyanat oder dem geeigneteren Butandiisocyanat) als reaktionsfähiges Gemisch auf die Außenseite des Vlieses aufgebracht und getrocknet. Durch eine Additionsreaktion des Isocyanates und eine teilweise hieraus erfolgende Vernetzung wird eine geschlossene hydrogelbildende Beschichtung mit einer Schichtdicke von etwa 10 µm bis etwa 200 µm gebildet. Beim Kontakt mit Wasser bildet sich auf der Außenseite des Implantats ein Hydrogel, welches eine attraktive Wechselwirkung mit der Gefäßwand aufweist und das Vlies des Implantats an der Gefäßwand fixiert. Somit wird die Flap-Bildung verhindert und die Gefahr einer Thrombosenbildung durch das Implantat reduziert bzw. vermieden. Ein weiterer Vorteil einer hydrogelen Beschichtung ist, dass diese die Biokompatibilität der Membran positiv beeinflussen kann. So ist zum Beispiel mit Hydrogelen auf der Basis von Polyethylenglykol die Fremdkörperreaktion so weit unterdrückbar, dass nahezu keine Reaktion mehr detektierbar ist.

Es ist weiterhin von Vorteil, wenn das Befestigungsmittel durch vorzugsweise axial verlaufende Versteifungselemente gebildet ist, mit denen die Grundstruktur in dem jeweiligen Abschnitt versehen ist. Der Vorteil dieser Versteifungselemente besteht darin, dass das Vlies besser befestigt wird, so dass Flaps vermieden werden und damit die Thrombosengefahr vermindert wird. Gegenüber der Aufnahme zusätzlicher Stege in die Grundstruktur besitzt dieses Ausführungsbeispiel den zusätzlichen Vorteil, dass die Stege nachträglich an jeder beliebigen Stelle der Grundstruktur befestigt werden können. Das Implantat kann somit an die besonderen Bedürfnisse während der Behandlung optimal angepasst werden.

Die Versteifungselemente enthalten vorzugsweise mindestens ein Metall und/oder ein Material, das einen hohen Elastizitätsmodul besitzt, und/oder ein Material, das mindestens eine deutlich höhere Steifigkeit als das das Vlies bildende Polymer besitzt. Prinzipiell sind metallische und polymere bioabbaubare biokompatible Materialien ebenfalls geeignet. In einem bevorzugten Ausführungsbeispiel eines Herstellungsverfahrens für ein Implantat mit Versteifungselementen wird die Grundstruktur zuerst durch einen Spinnprozess mit einer sehr dünnen Vliesschicht beschichtet. Die Versteifungselemente werden dann auf der Vliesschicht platziert und fixiert. Dies kann zum Beispiel dadurch realisiert werden, dass die Versteifungselemente zunächst mit einer der Spinnlösung ähnlichen Polymerlösung benetzt werden. Danach werden die einzelnen Versteifungselemente auf der beschichteten Grundstruktur in noch nicht ausgehärtetem Zustand der Benetzungsschicht positionsgerecht abgelegt, wodurch die Versteifungselemente spontan mit der Beschichtung verklebt werden. Nachdem die Elemente platziert sind, wird eine weitere Vliesschicht auf den Verbundkörper gesponnen. In einem anschließenden Verdichtungsprozess (unten beschrieben) wird dann ein geschlossener Materialverbund erzeugt. Durch die axialen Versteifungselemente wird die Gefahr der Bildung von Flaps ebenfalls verringert. Die Länge eines derartigen Versteifungselements beträgt vorzugsweise 1 mm bis mehrere Millimeter, Breite und Höhe betragen vorzugsweise weniger als 0,5 mm, besonders bevorzugt weniger als 0,1 mm. Vorzugsweise bilden die Versteifungselemente jeweils eine flache Quaderform aus. Beispielsweise bestehen die Versteifungselemente aus Edelstahl 316L, mit einer Höhe kleiner als 0,05 mm, einer Breite von etwa 0,1 mm und einer Länge von etwa 2 mm.

Um das Vlies zu spannen, kann in einem bevorzugten Ausführungsbeispiel als Befestigungsmittel ein elastisches Element vorgesehen werden, vorzugsweise ein elastischer Ring. Ein solches elastisches Element wird vorzugsweise aus dem Vlies durch einen nachträglichen Behandlungsschritt erzeugt. Es ist demnach in dem jeweiligen Abschnitt fest mit dem Vlies verbunden. Das E-Modul eines derartigen elastischen Elements beträgt vorzugsweise etwa 10 MPa bis etwa 40 MPa, die maximale Dehnung ist vorzugsweise größer als 300%.

Das Vlies wird mittels Elektrospinnen hergestellt. Beim Elektrospinnen wird eine Polymerlösung, vorzugsweise eine Lösung mit einem oben angegebenen Vlies-Polymer, durch eine Metalldüse gefördert. Zwischen der Metalldüse und der Grundstruktur liegt eine Hochspannung an. Die Grundstruktur liegt dabei auf einem anderen elektrischen Potential als die Metalldüse. Durch das Fördern der Polymerlösung und der Spannungsdifferenz zwischen der Düse und der Grundstruktur wird ein Endlos-Faden als Vlies auf die Grundstruktur aufgebracht.

Es ist weiterhin von Vorteil, wenn das Vlies in dem mindestens einen Abschnitt derart behandelt ist, dass es eine größere Dichte aufweist, d.h. verdichtet ist. Eine größere Dichte des Vlieses kann beispielsweise durch eine mechanische Behandlung, eine thermische Behandlung und/oder durch eine Behandlung mit Lösungsmittel und/oder Lösungsmitteldampf erreicht werden. Eine solche Behandlung, die im Folgenden auch als Verdichtungsprozess bezeichnet wird, verringert die Wahrscheinlichkeit, dass sich Fasern oder Faserabschnitte ablösen oder von dem Vlies abstehen. Zusätzlich wird durch die Verdichtung die Druckfestigkeit des Vlieses erhöht und Porosität beeinflusst.

Die Grundstruktur eines derartigen Implantats kann vorzugsweise ein Metall oder eine Metalllegierung, vorzugsweise Edelstahl, CoCr, eine Magnesiumlegierung (Implantat gestaltet als Stent bestehend aus einer Magnesiumlegierung wird auch AMS = absorbierbarer Metallstent genannt) und/oder Nitinol, und/oder ein Polymer aus der Klasse der bioabbaubaren Polymere, vorzugsweise Polymilchsäuren, Poly-caprolactone und/oder Mischungen oder Copolymere daraus, und/oder ein Polymer aus der Klasse der biokompatiblen Polymeren, vorzugsweise UHMWPE und/oder PEEK, enthalten. In einem weiteren Ausführungsbeispiel kann eine metallische Grundstruktur zusätzlich mit einer Beschichtung aus amorphem Siliziumkarbid (aSiC-Beschichtung) versehen sein.

Die obige Aufgabe wird auch durch ein Verfahren zur Herstellung eines Implantats mit den folgenden Schritten gelöst:
- Bereitstellen einer Grundstruktur,
- Anordnen mindestens eines ersten Teils eines Vlieses auf und/oder in der Grundstruktur, mittels Elektrospinnen,
- Einbringen und/oder Aufbringen eines Befestigungsmittels am proximalen und/oder am distalen Ende des Implantats, derart, dass zumindest in einem expandierten Zustand des Implantats ein Abragen von Vliesabschnitten von der Grundstruktur und/oder die Bildung von größer ausgedehnten Bereichen, in denen Fasern oder Faserabschnitte von der Grundstruktur abragen, signifikant verringert wird,
- gegebenenfalls Anordnen eines zweiten Teils des Vlieses auf und/oder in der Grundstruktur, mittels Elektrospinnen,
- gegebenenfalls Befestigen des Implantats auf einem Katheter, vorzugsweise auf einem Ballon eines Katheters.

Ein derartiges Verfahren ist einfach, um einerseits ein Implantat herzustellen, welches auf Grund seiner Porosität gut endothelialisiert wird. Andererseits weist das Implantat eine reduzierte Gefahr der Thrombosenbildung auf, da, wenn das Vlies nach dem erfindungsgemäßen Verfahren hergestellt wird, Teile des Vlieses (Fasern oder Faserabschnitte) daran gehindert werden, in das Lumen, in dem die Körperflüssigkeit fließt, hineinzuragen.

Insbesondere dann, wenn das Befestigungsmittel, vorzugsweise ein oben beschriebenes, insbesondere axial verlaufendes Versteifungselement, in das Vlies eingebettet werden soll, ist es vorteilhaft, wenn zunächst ein erster Teil des Vlieses auf der Grundstruktur angeordnet wird, anschließend das Befestigungsmittel in einem Abschnitt des Implantats auf und/oder in dem Vlies angeordnet wird und danach ein zweiter Teil des Vlieses auf der mit dem erstem Teil des Vlieses und dem Befestigungsmittel versehenen Grundstruktur angeordnet wird.

In einer alternativen Ausführungsform kann jedoch auch das gesamte Vliesmaterial zunächst auf der Grundstruktur angeordnet werden und anschließend das Befestigungsmittel ein- und/oder aufgebracht werden und/oder das Vlies behandelt werden.

Die Porosität des Vlieses kann durch die Parameter des Elektrospinnens und durch die Parameter eines Verdichtungsprozesses gesteuert werden und somit auch die Endothelialisierung bzw. die Blutdurchlässigkeit und die Druckfestigkeit des Vlieses. Aufgrund des elastischen Verhaltens des Vlieses wird die Steifigkeit der Grundstruktur nur unwesentlich verändert, was weder zu einem zusätzlichen Beitrag zum Recoil der Grundstruktur noch zu einer erhöhten Restenose-Rate aufgrund einer erhöhten Steifigkeit führt.

In einem besonders bevorzugten Ausführungsbeispiel wird das Vlies durch eine thermische Behandlung, eine mechanische Behandlung und/oder durch Wechselwirkung mit mindestens einem Lösungsmittel, einer Dispersion und/oder einer Klebstofflösung in dem mindestens einen Abschnitt verdichtet. Als Verdichtung wird im Folgenden eine Maßnahme bezeichnet, bei der ein Vlies intensiv vernetzt und fixiert wird. Das Vlies ist in dem mindestens einen Abschnitt stärker vernetzt als in den übrigen Abschnitten, in denen keine Verdichtung erfolgt ist.

Gegebenenfalls wird das Implantat nach der Verdichtung noch an einer vordefinierten Stelle abgetrennt, so dass beispielsweise eine bestimmte Länge des Implantats gewährleistet wird. Ferner kann vorzugsweise im Zusammenhang mit einer Abtrennung des Implantats an einer vordefinierten Stelle die Verdichtung so intensiv erfolgen, dass ein elastisches Element gebildet wird. Im Folgenden werden einige Möglichkeiten beschrieben, wie eine Verdichtung erreicht werden kann oder die Verdichtung so intensiv erfolgt, dass ein elastisches Element entsteht.

Eine bevorzugte thermische Behandlung zum Verdichten des Vlieses stellen ein Temperschritt oder mehrere Temperschritte, z.B. in einem Umluftofen, dar, welche durch eine Wahl der Temperatur unterhalb der Polymererweichung und der Zeit ein Zusammensintern der Fasern des Vlieses in dem betroffenen Abschnitt bewirken. Durch die Montage eines mit dem Vlies versehenen Implantats unterhalb eines Schrumpfschlauches kann eine glatte und homogene Oberflächenstruktur des Vlieses nach der Beendigung der thermischen Behandlung erreicht werden, welche durch den Schrumpfprozess die Faserstruktur mechanisch komprimiert.

Eine besonders bevorzugte und sehr produktive Variante der Verdichtung durch eine thermische Behandlung erfolgt mittels elektromagnetischer Strahlung. Durch elektromagnetische Strahlung können die Fasern des Vlieses über deren Eigenabsorption sehr kurz aufgeheizt werden. Hierbei sind die Wellenlängen der zur Verfügung stehenden Strahlung sehr wichtig, da sie über Absorption und Streuung innerhalb der Faser die Eindringtiefe in die Faser bestimmen. Monochromatische Laserstrahlung im NIR erzeugt bei den oben genannten Vlies-Polymertypen nur geringe Absorptionen und eine hohe Eindringtiefe und hierdurch eine homogene thermische Verdichtung der Faserstruktur.

Ein weiteres technisch interessantes Ausführungsbeispiel ergibt sich durch ungefärbte Additive, beispielsweise Clearweld®, die als Zusatzmaterial in dem Fasermaterial des Vlieses oder als Beschichtung auf dem Vlies die Nutzung von preiswerten und leistungsfähigen Feststoff- oder Diodenlasern im Wellenlängenbereich von 1100 nm bis ca. 750 nm als Strahlungsquelle zur Verdichtung zulassen. Die Verwendung eines CO₂-Lasers führt mit seiner wesentlich langwelligeren Strahlung und der häufig sehr hohen Eigenabsorption der genannten Polymere zu einer wenige µm dicken Verdichtung der bestrahlten Oberfläche. Diese Verdichtung durch Energieabsorption aus der Strahlung und wird durch die thermische Aufheizung der Strahlungsabsorption erzeugt. So lässt sich zum Beispiel eine elektrogesponnene Vliesschicht aus Pellethan mittels einer CO₂-Laserbestrahlung mit einer Oberfläche versehen, die aus einer sehr dünnen, umgeschmolzenen und dichten Polymerschicht besteht, welche die vliesartige Unterstruktur abdeckt. In diesem Ausführungsbeispiel wird durch geeignete Parameterwahl nur das Zusammenschmelzen der gesponnen Polymerfäden an der Oberfläche des Vlieses angestrebt.

Eine Verdichtung mittels elektromagnetischer Strahlung ist vor allem für die Ausbildung einer mechanisch stärker belastbaren Grenzschicht an der Außenseite des Vlieses vorteilhaft. So kann das außenliegende Vlies der beschichteten Grundstruktur insbesondere bei einem Stent in seinem auf den Katheter montierten Zustand besser gegen mechanische Belastungen, wie z.B. eine Passage durch eine hämostatische Schleuse, geschützt werden. Ferner kann eine höhere Grifffestigkeit für das Handling bei der Behandlung mit dem Implantat oder im Produktionsprozess erreicht werden. Generell ist der hohe Energieeintrag in die Oberfläche gut geeignet, abstehende Strukturen aufzuschmelzen und an der Vliesoberfläche zu fixieren. Diese Vorteile werden dadurch erreicht, dass in überraschender Weise die von dem Vlies abragenden Fasern bevorzugt aufgeschmolzen werden, sich bei der Erweichung intensiv verkürzen und somit sehr effektiv über einen Schmelzprozess mit anderen an der Oberfläche des Vlieses angeordneten Fasern verbunden werden.

Eine weitere Variante der Verdichtung ist die Wechselwirkung mit Lösungsmitteln. Durch sehr potente Lösungsmittel, wie dies z.B. THF für verschieden Polyurethane darstellt, oder Chloroform für milchsäure-basierte resorbierbare Polymere, wird ein Quellen des Vlies-Polymers erreicht. Dieser Prozess wird jedoch vor der vollständigen Lösung des Polymers unterbrochen. Dieses Quellen kann über die Aufnahme des Lösungsmittels aus der Gasphase (Verdichten mittels Lösungsmitteldampf) oder durch direkten Lösungsmittelkontakt erfolgen. Bei der Aufnahme des Quellungs- bzw. Lösungsmittels über die Gasphase kann der Sättigungsdampfdruck des genutzten Quellungsmittels über die Mischung mit anderen Lösemitteln eingestellt werden. Bei direktem Lösungsmittelkontakt kann die Kontrolle der Quellung zusätzlich durch die Zugabe von Nichtlösungsmitteln erfolgen. Ein solches System stellt z.B. für viele Polyurethane als Vliesmaterial ein Gemisch aus THF und Wasser dar.

Auch bei der Verdichtung durch Lösungsmittel kann eine deutliche Relaxation der gesponnenen Fasern des Vlieses beobachtet werden. Diese Relaxation führt dazu, dass die flächig abgelegte Faserstruktur eine Spannung aufbaut, sich zunehmend zusammenzieht und somit auch mechanisch verdichtet. Optisch ist dieser Prozess dadurch sichtbar, dass typischerweise die Fasern des Vlieses nach dem Spinnprozess die besponnene Grundstruktur umschmiegen. Nach dem Verdichtungsprozess spannt sich eine membranartige Faserstruktur zwischen den entsprechenden Elementen der Grundstruktur auf und delaminiert von der Grundstruktur in den Randbereichen der Grundstruktur. In diesem Prozess entstehen mehr Kontaktierungspunkte in der nach dem Spinnen noch losen Faserstruktur. Es ist auch zu beobachten, dass sich noch leicht abragende Faserstrukturen an die Oberfläche anschmiegen.

Weitere Lösungsmittel oder ein Lösungsmittelgemische bestehen aus Tetrahydrofuran (THF) oder Dimethylformamid (DMF) oder aus einer Mischung dieser Lösungsmittel (einzeln oder in Kombination mit Wasser oder niederen Alkoholen (Methanol, Ethanol, Isopropanol)).

Durch die beschriebenen Verdichtungsprozesse wird verhindert, dass Teile des Vlieses von der Grundstruktur abragen. Da der Spinnprozess die Faserstruktur potentiell aus einer kontinuierlichen Faser bereitstellt und die Verdichtungsmethode an Verbindungsstellen zu multiplen Fixierungen der Faser führt, ist gewährleistet, dass das mechanisch intakte flächenförmige Fasergebilde (Vlies) keinerlei Faserfragmente an die Umgebung abgeben kann.

Zum Verdichten und Abschneiden des Implantats wird dieses, das bereits mit dem Vlies versehen ist, beispielsweise auf einen Dorn montiert, welcher an einem rotierenden Motor angeschlossen ist. Danach wird das proximale und/oder distale Ende des Implantats mittels einer mechanischen Scherung, z.B. über eine rotierende Klinge, abgetrennt.

Eine interessante Verfahrensvariante ist der Einsatz eines fokussierten Laserstrahles zum Zweck der Abtrennung oder zur Perforation des Vlieses. Wie bei der Verdichtung beschrieben unterliegen die gesponnenen und orientierten Fasern vor dem Aufschmelzen einer starken Relaxation. Dieser Effekt kann genutzt werden, um unter Rotation des mit dem Vlies versehenen Implantates und lokaler axialer Erhitzung des Fasermateriales durch einen fokussierten Laserstrahl ein axiales Aufschmelzen des Fasermateriales und effektives radiales Zurückziehen dieser Schmelze zu erreichen.

Wie oben bereits dargestellt wurde, kann das Verdichten und/oder Abschneiden so intensiv erfolgen, dass aus dem Vliesmaterial in dem betreffenden Abschnitt ein elastisches Element, vorzugsweise ein elastischer Ring, gebildet wird, welches ebenfalls das Abragen von Fasern und Faserabschnitten in dem betreffenden Bereich verhindert.

Bei der oben beschriebenen Abtrennung des Vlieses mittels eines Laserstrahls, insbesondere bei Versuchen mit gesponnenen PUR Fasern, stellte sich heraus, dass abhängig von den Laserparametern die Relaxation der Vliesfaser(n) so stark ist, dass sich die Schmelze trennt. Eine besondere Eigenschaft dieser Trennungsstelle ist, dass die abgeschmolzenen Faserenden eine radiale Ansammlung von Polymer erzeugen, welches dann in einem disorientierten Zustand erstarrt. Dieses Verhalten trifft prinzipiell für alle thermoplastisch verarbeitbaren Polymere, nicht nur für TPU (thermoplastisches Polyurethan), zu. Durch die Breite des Laserstrahls und dessen geringe Verschiebung in axialer Richtung wird geschmolzenes Material an der Schmelzgrenze akkumuliert. Erreicht die Schmelzgrenze des Vlieses die Grundstruktur des Implantates, so wird das Vlies an diese Grundstruktur angeschmolzen. Nach dem Erstarren der Schmelze entsteht eine Polymerstruktur in nicht orientierter Form aus dem Vlies, die aus demselben Material wie die Vliesfasern selbst besteht. Ist die Elastizität des Polymers geeignet, die Verformungen zu dem Zieldurchmesser zuzulassen, entsteht somit ein elastisches Element in dem behandelten Bereich der Membran, welche durch die elastische Eigenspannung den jeweiligen Abschnitt des Vlieses gespannt hält. Hierbei wird als Zieldurchmesser der gewünschte Durchmesser des Implantats im dilatierten Zustand bezeichnet, wobei der Zieldurchmesser durch den Durchmesser des Gefäßes vorgegeben wird, in welches das Implantat implantiert wird. Dieser Effekt kann genutzt werden, um einer Taschenbildung des Vlieses im Blutstrom entgegenzuwirken, da der Bereich mit dem elastischen Element eine höhere radiale Spannung aufbaut als das unbehandelte Vlies. Im Bereich des elastischen Elements werden ferner die Faserenden des Vlieses fixiert. Wenn sich das elastische Element um den gesamten Umfang der Grundstruktur herum erstreckt, wird das elastische Element auch als elastischer Ring bezeichnet.

Alternativ kann ein Abschnitt des mit dem Vlies versehenen Implantats, vorzugsweise das distale oder proximale Ende, senkrecht auf ein mit Lösungsmittel oder mit einer Polymerlösung, die vorzugsweise dem Polymer des Vlieses entspricht, getränktes Substrat gedrückt werden. Dadurch werden die in dem jeweiligen Abschnitt abragenden Teile des Vlieses definiert aufgelöst und somit abgelängt sowie an den jeweiligen Abschnitt der Grundstruktur angebunden. Die durch die Behandlung mit Lösungsmittel oder Polymerlösung erfolgende Verfilmung und die hierdurch prinzipiell erreichbaren Vorteile entsprechen den vorausgegangenen Erläuterungen der Laserbehandlung zur Erzeugung des elastischen Elements, da durch die Verfilmung eine unorientierte Polymeransammlung, eine Fixierung randständiger Fasern und eine Benetzung der Grundstruktur erfolgt. Ein grundsätzlicher Unterschied der Laserbehandlung zu der Behandlung mit einem Lösungsmittel oder einer Polymerlösung besteht darin, dass durch die Behandlung mit einem Lösungsmittel oder einer Polymerlösung effektiv Material abgelöst und entfernt wird. Dies ergibt eine deutlich dünnere, d.h. filigranere, Materialansammlung im jeweiligen Abschnitt des Implantates und somit ein dünneres elastisches Element.

Alternativ kann ein elastisches Element auch über eine thermische Behandlung (beispielsweise mittels eines Heizelements, eine induktive Heizung, IR-Strahlung und/oder Licht) an dem jeweiligen Abschnitt des Implantats durch Verschmelzen der Faserstruktur des Vlieses erzeugt und an der Grundstruktur befestigt werden.

Mit anderen Worten kann durch eine entsprechende Behandlung des Implantats in einem Bereich des Vlieses derart, dass das Vliesmaterial bis zur Grundstruktur aufschmilzt, dort akkumuliert und erstarrt, wobei das geschmolzene und anschließend wieder erstarrte Polymer eine gewisse Elastizität aufweist, ein elastisches Element aus dem Vlies erzeugt werden. Hierbei muss die Elastizität beispielsweise im Falle eines Stentgrafts ausreichend sein, um die Verformung der bearbeiteten Struktur auf den gecrimpten Durchmesser (gecrimpter Zustand) und die radiale Expansion des Stentgrafts dauerhaft und ohne Bruch zu erlauben.

Ein weiteres Ausführungsbeispiel des erfindungsgemäßen Verfahrens sieht vor, dass an der Außenseite des Vlieses ein Hydrogel und/oder ein hydrogelbasierter Klebstoff angeordnet werden. Der Mechanismus, durch den eine Entstehung von Flaps verhindert wird, ist weiter oben erläutert.

In einem weiteren Ausführungsbeispiel wird in dem mindestens einen Abschnitt innen in das Implantat mindestens eine Torsionsfeder eingesetzt und/oder auf die Außenseite mindestens eine Torsionsfeder aufgesetzt und/oder es werden in dem mindestens einen Abschnitt in das Vlies vorzugsweise axial verlaufende Versteifungselemente eingebettet. Ein Ausführungsbeispiel zur Einbettung der Versteifungselemente in das Vlies ist weiter oben beschrieben.

Ebenfalls bevorzugt ist es, wenn das Vlies zumindest in dem mindestens einen Abschnitt mittels Laser perforiert wird. Dieses Verfahren hat den Vorteil, dass bei gleich bleibender Druckfestigkeit eine höhere Porosität, z. B. zur Abdeckung von Aneurysmen, des Vlieses erreicht werden kann. Derart hergestellte Implantate werden insbesondere in der Neurologie eingesetzt.

Die Porosität oder Morphologie des Vlieses kann durch geeignete Auswahl der Spinnparameter (z.B. den Abstand der geladenen Düse zur Grundstruktur (Target), die Spinntemperatur, die Luftfeuchtigkeit, die Lösungsmitteldampfkonzentration in der Spinnapparatur, Hochspannungsdifferenz zwischen Düse und Grundstruktur und/oder die Art des Lösungsmittels oder Lösungsmittelgemisches) gezielt gesteuert werden. Zusätzlich kann die Porosität und die Morphologie über die Konzentration der Polymerlösung und die Durchflussrate beeinflusst werden. Durch einen zusätzlichen Eintrag von Wärmestrahlung kann eine vorzugsweise axiale Ausrichtung der Faser(n) des Vlieses auf der Grundstruktur oder dem Ballon erzielt werden.

Das erfindungsgemäße Verfahren bzw. das erfindungsgemäße Implantat wird nachfolgend in Beispielen anhand von Figuren erläutert. Dabei bilden alle beschriebenen und/oder bildlich dargestellten Merkmale den Gegenstand der Erfindung, auch unabhängig von ihrer Zusammenfassung in den Ansprüchen oder deren Rückbezüge.

### Es zeigen schematisch:

- Fig. 1: ein nach dem Stand der Technik hergestellter Stentgraft in einer Querschnittdarstellung,
- Fig. 2: ein weiterer nach dem Stand der Technik hergestellter Stentgraft in einer Querschnittdarstellung (Figur 2a) und in einer fotografischen Aufnahme von der Seite (Figur 2b),
- Fig. 3: ein erstes Ausführungsbeispiel eines erfindungsgemäßen Stentgrafts in einer Querschnittdarstellung,
- Fig. 4: ein zweites Ausführungsbeispiel eines erfindungsgemäßen Stentgrafts in einer Querschnittdarstellung,
- Fig. 5: ein drittes Ausführungsbeispiel eines erfindungsgemäßen Stentgrafts in einer Querschnittdarstellung (Figur 5c) sowie einen Abschnitt der Grundstruktur dieses Ausführungsbeispiels in einer Ansicht von der Seite im Vergleich mit einem Stentgraft nach dem Stand der Technik in einer Querschnittdarstellung (Figur 5a) und einer Ansicht der Grundstruktur von der Seite (Figur 5b),
- Fig. 6: ein viertes Ausführungsbeispiel eines erfindungsgemäßen Stentgrafts in einer Querschnittdarstellung,
- Fig. 7: ein fünftes Ausführungsbeispiel eines erfindungsgemäßen Stentgrafts in einer Ansicht von der Seite,
- Fig. 8: ein sechstes Ausführungsbeispiel eines erfindungsgemäßen Stentgrafts in einer Querschnittdarstellung und
- Fig. 9: das sechste Ausführungsbeispiel gemäß Figur 8 in zwei fotografischen Aufnahmen von der Seite (Figuren 9a und 9c) sowie in einer fotografischen Aufnahme von vorn (Figur 9b).

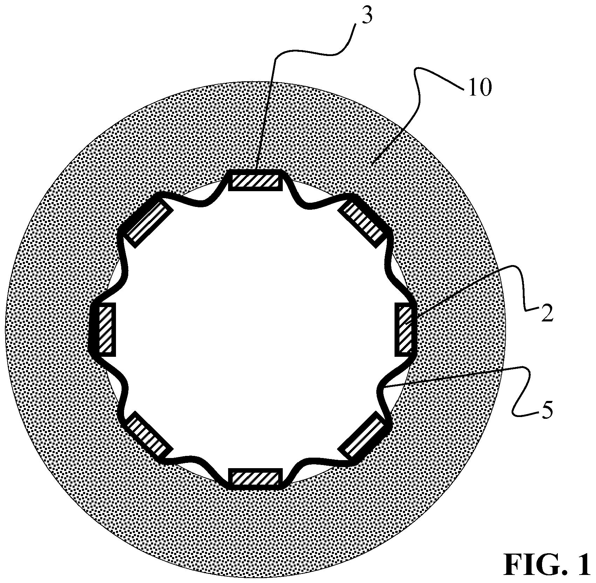

Figur 1 zeigt einen nach dem Stand der Technik hergestellten Stentgraft im dilatierten Zustand, welcher innerhalb eines Blutgefäßes 10 angeordnet ist. Das Vlies 3 des Grafts ist auf der äußeren Mantelfläche der Grundstruktur, deren Stege 2 in der Abbildung sichtbar sind, angeordnet. In der Figur 1 ist gut erkennbar, dass das Vlies 3 Flaps 5 ausbildet, welche aufgrund des durch die Flussgeschwindigkeit des Blutes entstehenden Drucks in das behandelte Blutgefäß 10 hinein gezogen werden und hierdurch den Durchfluss des Blutes behindern.

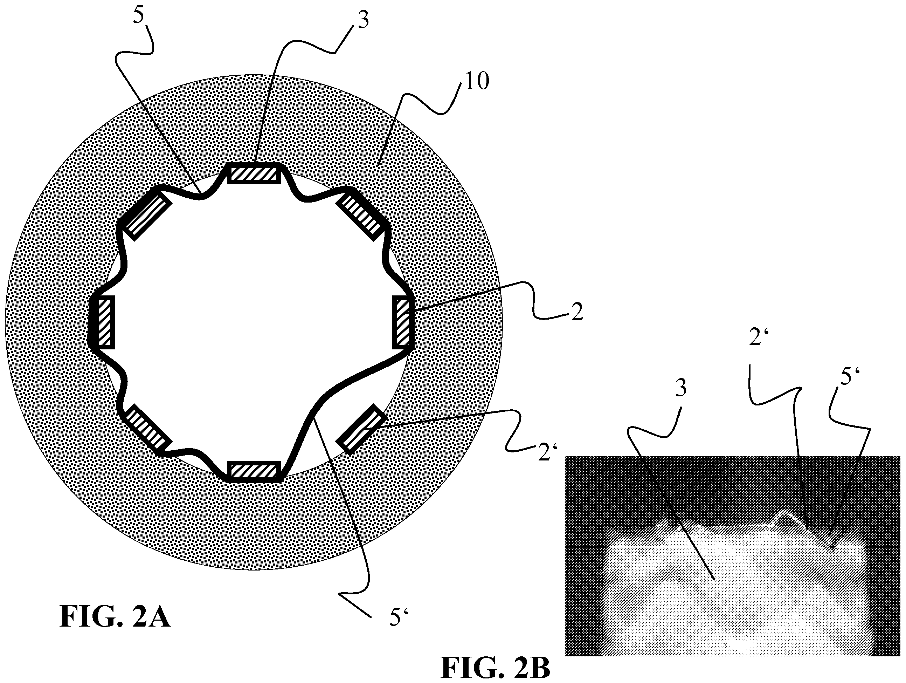

In Figur 2 ist zu sehen, dass sich die Flapbildung bei einem nach dem Stand der Technik hergestellten Stentgraft sogar über zwei Maschen der Grundstruktur erstrecken kann. Der Flap 5' ist durch Ablösung des Vlieses 3 von dem Steg 2' entstanden und bildet eine sehr große Tasche, die den Blutdurchfluss in dem Blutgefäß 10 sehr stark behindert. Der Flap 5' ist in der fotografischen Abbildung in Figur 2b) deutlich erkennbar.

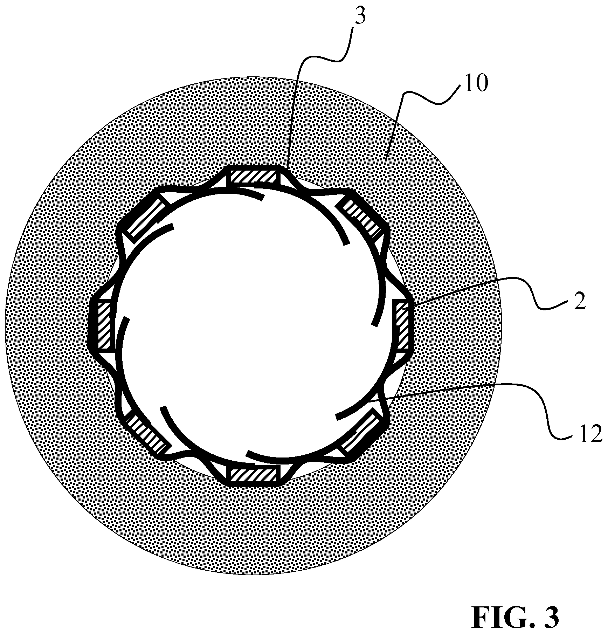

Das in Figur 3 dargestellte erste Ausführungsbeispiel eines erfindungsgemäßen Stentgraft weist eine an der inneren Oberfläche des Stentgraft angeordnete Torsionsfeder 12 auf, welche durch den von ihr nach außen wirkenden Druck dafür sorgt, dass sich Flaps nicht bilden oder keine größer ausgedehnten Bereiche mit Fasern oder Faserabschnitten in das Innere des Gefäßes 10 abragen. Das in Figur 3 dargestellte Vlies 3 kann neben der dargestellten Anordnung auf der äußeren Mantelfläche der Grundstruktur auch an der Innenseite der Grundstruktur mit den Stegen 2 angeordnet sein.

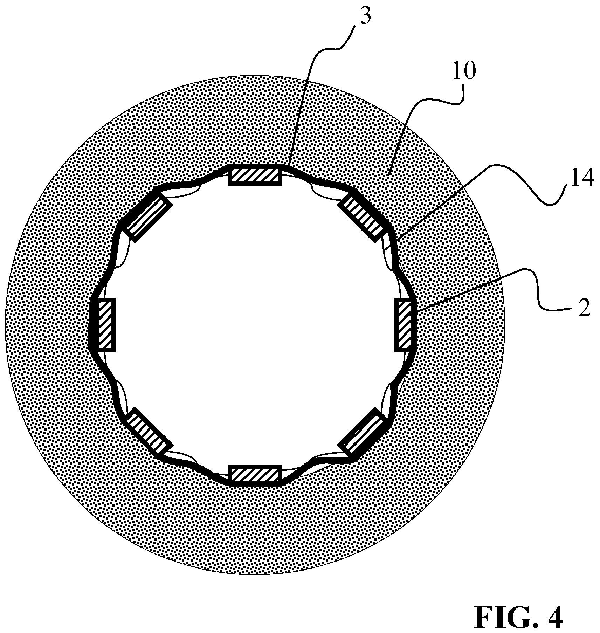

Figur 4 zeigt das zweite Ausführungsbeispiel eines erfindungsgemäßen Stentgrafts, wobei auch in dieser Darstellung ein an der Innenseite der Grundstruktur mit den Stegen 2 angeordnetes Vlies 3 nicht gezeigt ist, sondern nur die Anordnung des Vlieses 3 auf der Außenseite. Die Anordnung des Vlieses 3 an der Innenseite der Grundstruktur ist alternativ jedoch auch möglich. Als Befestigungsmittel weist die Grundstruktur im Wesentlichen in Umfangsrichtung verlaufende Konnektoren 14 auf, welche die Stege 2 zusätzlich miteinander verbinden. Jeder Konnektor 14 besteht aus dem gleichen Material wie die Grundstruktur und kann eine S-Form aufweisen.

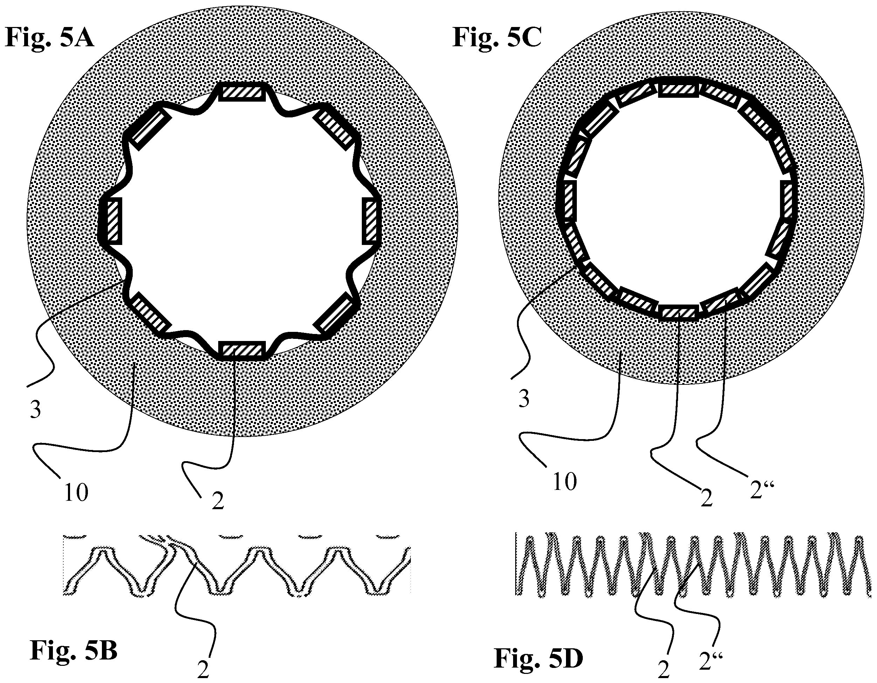

In Figur 5c) und 5d) wird das dritte Ausführungsbeispiel eines erfindungsgemäßen Stentgrafts dargestellt, welches in einem Abschnitt eine größere Dichte an Stegen 2, 2" aufweist. Die größere Dichte an Stegen 2, 2" wird in den Abschnitten vorgesehen, welche besonders anfällig für eine erhöhte Thrombosen-Gefahr sind, vorzugsweise am proximalen und/oder am distalen Ende des Implantats. Dort wird das Vlies an die Grundstruktur so angebunden, dass sich das Vlies, lose Fasern oder Faserabschnitte insbesondere bei der Zuführung, Positionierung und Freisetzung des Implantats sowie während der vorgesehenen Funktionsdauer nicht von der Grundstruktur ablösen können. In den übrigen Abschnitten des Stentgrafts entspricht die Dichte der Stege 2 dem Stentgraft nach dem Stand der Technik, welcher zum Vergleich in den Figuren 5a) und 5b) dargestellt ist. Auch in diesen Darstellungen ist jeweils ein an der Innenseite und/oder der Außenseite der Grundstruktur mit den Stegen 2 bzw. 2, 2" angeordnetes Vlies nicht gezeigt.

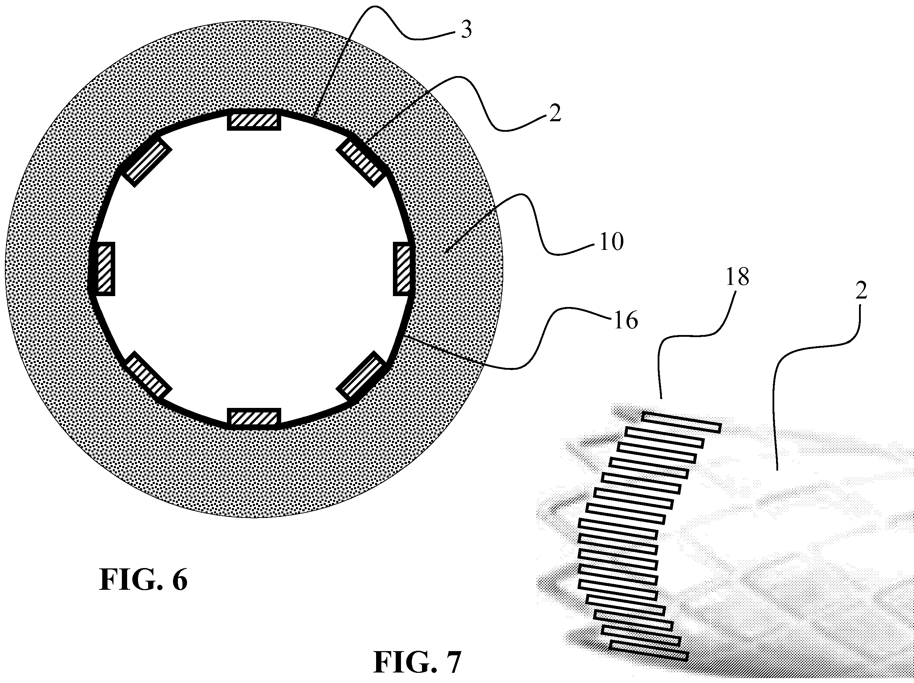

Das in Figur 6 dargestellte vierte Ausführungsbeispiel eines erfindungsgemäßen Stentgrafts zeigt als Befestigungsmittel eine auf dem gesamten Umfang des Stentgrafts vorgesehene Beschichtung 16, welche an der Außenseite, an der Innenseite und/oder der Außenseite der Grundstruktur mit den Stegen 2 angeordneten Vlieses aufgebracht ist. Die Beschichtung 16 enthält ein Material, das ein Hydrogel und/oder einen hydrogelbasierten Klebstoff bildet. Die Beschichtung 16 mit dem Hydrogel wird durch Wasseraufnahme aus dem Blut des Blutgefäßes 10 gequollen, so dass eine attraktive Wechselwirkung zwischen der äußeren Oberfläche des Stentgrafts (d.h. der äußeren Oberfläche des Vlieses) und der Wand des Blutgefäßes 10 entsteht. Hierdurch wird der Stentgraft förmlich an das Blutgefäß 10 geklebt.

Figur 7 zeigt ein fünftes Ausführungsbeispiel eines erfindungsgemäßen Stentgrafts. Bei diesem Ausführungsbeispiel ist das Befestigungsmittel durch axial verlaufende Versteifungselemente 18 gebildet, mit denen die Grundstruktur mit den Stegen 2 in dem jeweiligen Abschnitt versehen ist. Der Vorteil dieser Versteifungselemente 18 besteht darin, dass das Vlies an den distalen und proximalen Enden des Stentgrafts versteift wird, so dass Flaps vermieden werden und damit die Thrombosengefahr vermindert wird. Die Versteifungselemente 18 sind vorzugsweise in das Vliesmaterial eingebettet.

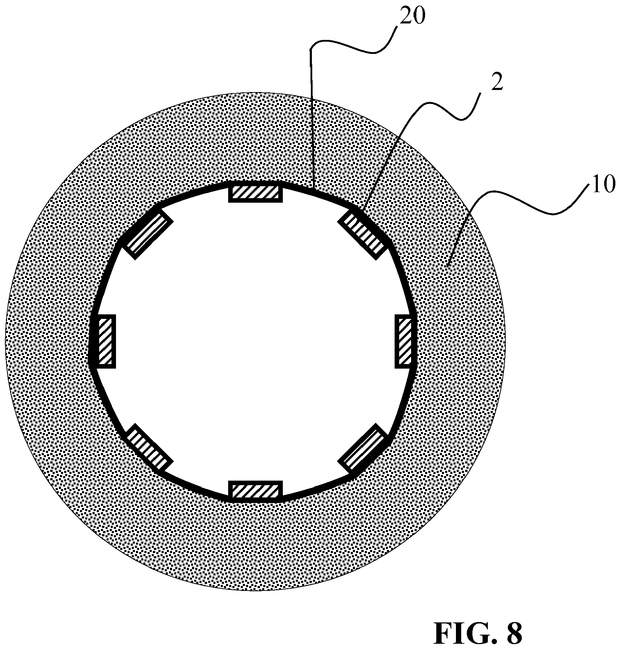

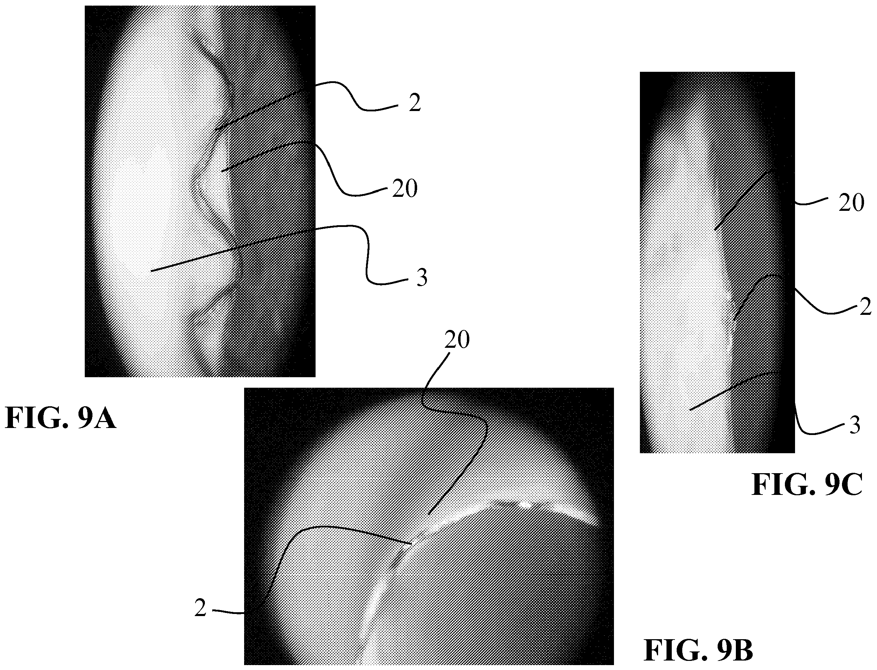

Das letzte, in den Figuren 8 und 9 dargestellte sechste Ausführungsbeispiel weist an dem distalen und/oder proximalen Ende des Stentgrafts einen elastischen Ring 20 auf, welcher die Bildung von Flaps verhindert. Der elastische Ring 20 wurde, wie unten detailliert beschrieben wird, durch Verdichtung aus dem Material des in Figur 8 dargestellten Vlieses 3 gebildet. Dies ist den in Figur 9 gezeigten Fotografien deutlich entnehmbar.

Ein erfindungsgemäßer Stentgraft wird vorzugsweise mittels der nachfolgend dargestellten Verfahren hergestellt.

Zunächst wird eine Polymerlösung aus Granulat und Dimethylformamid (DMF) angesetzt, wobei die Polymerlösung vorzugsweise biokompatibles thermoplastisches Polyurethan (TPU) in einer Konzentration von 15 Gew.% bis 25 Gew.% enthält. Die Polymerlösung wird in einer Apparatur mit Temperatur- und Klima-Überwachung bzw. -Regelung (Temperatur 20°C - 40°C, weniger als 30% relative Luftfeuchtigkeit) mittels einer Spritzenpumpe zu einer Metalldüse geführt, welche an einer Hochspannung anliegt. Die Durchflussrate liegt dabei in einem Bereich von minimal 0.1 ml/h bis maximal 10 ml/h. Das Grundgitter des Stents wird auf einen leitfähigen Dorn aufgezogen, welcher geerdet ist und sich in einem variierenden Abstand von 80 mm bis 250 mm vor der Düse dreht. Ferner ist eine IR-Lampe vorgesehen, welche den von der Metalldüse auf das Grundgitter aufgrund der anliegenden Hochspannung gezogenen endlosen Faden trocknet. Der getrocknete endlose Faden bildet das Vlies.

Anschließend kann der Stent mit dem Vlies verdichtet werden. Der verdichtete Stent wird anschließend mittels Laser und/oder Lösungsmittel und/oder thermischer Behandlung abgeschnitten und fixiert. Anschließend kann der Stentgraft auf ein SDS gecrimpt werden (SDS = stent delivery system). Mittels eines derartigen Verfahrens wird ein Stentgraft hergestellt, welcher ein verdichtetes Vlies insbesondere auf seiner Außenseite aufweist.

Eine Verdichtung eines nach obigem Verfahren hergestellten Stentgrafts mit TPU-Vlies kann dadurch erfolgen, dass der Stent mit dem Vlies auf ein PTFE Röhrchen aufgeschoben wird und eine Lage PTFE-Folie um das Implantat gewickelt wird. Anschließend wird ein Schrumpfschlauch über die PTFE-Folie gezogen. Die Kombination aus Stentgraft, PTFE-Röhrchen und PTFE-Folie mit Schrumpfschlauch wird dann auf ein Metallröhrchen geschoben und zusammen mit dem Metallröhrchen in eine Heizbackenmaschine eingelegt. Nun wird in der Maschine eine Temperatur von 200°C bis 220°C für 8 Sekunden erzeugt. Danach wird der thermisch behandelte Stentgraft aus der "Packung" gelöst. Durch die thermische Behandlung mit Schrumpfschlauch wurde eine Verdichtung des Vlieses erreicht, wobei durch den Schrumpfschlauch zusätzlich eine glatte und homogene Vlies-Mantelfläche erzeugt wurde. Anstelle einer Heizbackenmaschine kann auch eine induktive Heizquelle verwendet werden.

Eine weitere interessante Verfahrensvariante ist die Montage des besponnenen und optional bereits verdichteten Implantats nach dem Ablängen (z.B. durch Abrollen einer Klinge über einen auf einem Dorn angeordneten und dort besponnen Stentgraft) an dessen Enden auf einem rohrförmigen oder auch massiven Dorn. Durch Heizbacken, welche diesen Dorn in einem Abstand von 0 bis 2 mm vom Ende des Implantats greifen, kann durch einen intensiven und zeitlich begrenzten Wärmeimpuls die Strahlungswärme und/oder die Wärmeleitung des Dornes genutzt werden, um ein Schmelzen des Vlieses und die oben beschriebene Bildung eines elastischen Elements, insbesondere eines elastischen Rings, im Randbereich des Implantates zu erzeugen. Typische Verfahrensparameter sind z.B. die Platzierung des Stentgrafts mit einem Innendurchmesser von ca. 1,1 mm auf einem Edelstahlrohr mit den Maßen Außendurchmesser 1,1 mm und Innendurchmesser 0,95 mm. Ein 0,5 bis 2 Sekunden anhaltender Wärmeimpuls durch ein Erfassen des Edelstahlrohres mit den Backen eines Backenschweißers mit einer Temperatur der Heizbacken von etwa 240°C bis etwa 300°C (bei der Verwendung mittels z.B. Pellethane) und einer Länge der Heizbacken im Bereich von etwa 2 mm bis etwa 5 mm im Abstand von ungefähr 0,2 mm von dem distalen oder proximalen Ende des Stentgrafts führt zu einer sehr effektiven Verschmelzung des Vlieses in dem behandelten Abschnitt und der Bildung des elastischen Elements (siehe beispielsweise elastischer Ring 20 des in den Figuren 8 und 9 dargestellten sechsten Ausführungsbeispiels eines erfindungsgemäßen Stentgrafts), wobei durch diese Behandlung zusätzlich ein sich entlang der Längsrichtung der Grundstruktur fortsetzendes, aber in Intensität stetig abnehmendes Anschmelzen des Vlieses an die Grundstruktur des Implantats erreicht wird. Als besonders vorteilhaft wird bei dieser Technik die Ausbildung des Verbundes des Grundkörpers zu der Faserstruktur empfunden, da keine lokalisierte Verbindung im Randbereich sondern eine stetig zunehmende Verbindung zu behandelten Abschnitt des Implantats erfolgt. Dies wirkt sich potentiell günstig auf die Entstehung von Spannungsspitzen im Verbund des Grundkörpers mit der bedeckenden Faserstruktur aus und ermöglicht eine gute Kombination aus hohen Verformungsgrenzen und sicherer Fixierung der Faserstruktur auf der Grundstruktur des Implantats.

Alternativ kann die Verdichtung des Vlieses mittels CO₂-Laser erfolgen. Typische Parameter hierfür sind eine relative Leistung des CO₂-Lasers von ca. 6-20 W bei einem Laserfocus von ca. 1 mm², eine Rotation des zylinderförmigen besponnenen Implantates von ca. 100 - 300 Umdrehungen pro Minute und einer axiale Führungsgeschwindigkeit des Implantates von etwa 6 bis 10 mm/Sekunde. Als axiale Führungsgeschwindigkeit wird dabei die Geschwindigkeit des Implantats bezeichnet, mit der es sich in radialer Richtung an der feststehenden Laserquelle vorbeibewegt. Die angegebenen Parameter beschreiben einen praktikablen Betriebspunkt, tatsächlich können die Parameter Strahlungsleistung und Flächenvorschub sich gegenseitig ausgleichen, um die benötigte Schmelzenergie bereit zu stellen. Der Prozess ist technisch sehr gut steuerbar und kann für eine oberflächliche Verdichtung, welche die poröse Faserstruktur erhält, oder auch für eine vollständige Verfilmung zur Herstellung eines elastischen Elements oder alle Stufen zwischen diesen beiden Extremen verwendet werden.

Im Folgenden wird eine Verdichtung eines PU-Vlieses mittels eines Lösungsmittels beschrieben. Als praktikable Verfahrensparameter für THF-lösliche Polyurethane hat sich die Konditionierung des elektrogesponnenen Vlieses über einen Zeitraum von weniger als zwei Minuten in gesättigter THF-Dampfphase bei Raumtemperatur erwiesen. Es wurde festgestellt, dass die Verfahrensparameter zusätzlich von dem Restlösemittelgehalt, welcher nach dem Spinnprozess vorliegen kann, abhängig sind. Durch die Verwendung von Lösungsmittelgemischen, welche den Dampfdruck des quellenden Lösungsmittels verringern, und eine Variation der Temperatur lässt sich die Homogenität der Vernetzung der Faserstruktur des Vlieses beeinflussen. Die Verwendung eines Gemisches von THF und Wasser (mit einem Verhältnis THF/H₂O 100%/0% bis 30%/70% (jeweils in Vol%), bei Raumtemperatur) verdoppelt ungefähr die wählbare Konditionierzeit des Vlieses, wobei aufgrund von Diffusionseffekten die Faserstruktur tiefer und homogener gequollen werden kann.

Alternativ zu der oben dargestellten Vliesbildung direkt auf der Oberfläche der Grundstruktur können eine Faser oder mehrere Fasern auch auf eine geerdete Substratfläche (z.B. Tyvek-Folie) aufgetragen werden, welche in einem definierten Abstand vor der Düse platziert ist. Das elektrogesponnene Vlies wird anschließend auf einen Ballon aufgewickelt. Der Ballon mit dem aufgewickelten Vlies wird nun in das Innenlumen eines Grundgitters eines Stents platziert. Anschließend wird der Ballon expandiert und das Vlies hierdurch an der Innenseite des Grundgitters fixiert. Anschließend wird der Ballon in dem zu behandelnden Gefäß dilatiert und aus dem Stentgraft herausgezogen. Prinzipiell können für ein Stentgraft, das an der Innenseite ein Vlies aufweist, ebenfalls die oben dargestellten Verfahren zur Vermeidung von abragenden Fasern, Faserabschnitten oder Vliesabschnitten angewendet werden.

Die beiden oben angegebenen Verfahren, welche das Aufbringen von Vlies auf die Außenseite bzw. die Innenseite beinhalten, können auch kombiniert werden.

## Patentansprüche

1. Implantat ausgebildet als intraluminale Endoprothese mit einer hohlzylinderförmigen Grundstruktur, wobei in und/oder auf der Grundstruktur ein Vlies (3) angeordnet ist, welches biokompatibles thermoplastisches Polyurethan und/oder ein Polymer aus der Familie der Polymilchsäure oder Poly-caprolactone oder deren Copolymere und/oder ein Polymer aus der Familie der Polyphosphazene enthält, wobei in mindestens einem Abschnitt des Implantats am proximalen und/oder am distalen Ende des Implantats ein Befestigungsmittel 20 derart vorgesehen ist, dass zumindest in einem expandierten Zustand des Implantats ein Abragen von Vliesabschnitten von der Grundstruktur und/oder die Bildung von größer ausgedehnten Bereichen, in denen Fasern oder Faserabschnitte von der Grundstruktur abragen, verringert wird, **dadurch gekennzeichnet, dass** das Befestigungsmittel durch ein elastisches Element (20), vorzugsweise einen elastischen Ring, gebildet ist.

2. Implantat nach einem der vorhergehenden Ansprüche, **dadurch gekennzeichnet, dass** das Vlies mittels Elektrospinnen hergestellt ist.

3. Implantat nach einem der vorhergehenden Ansprüche, **dadurch gekennzeichnet, dass** das Vlies in dem mindestens einen Abschnitt derart behandelt ist, dass es eine größere Dichte aufweist.

4. Verfahren zur Herstellung eines Implantats ausgebildet als intraluminale Endoprothese mit einer hohlzylinderförmigen Grundstruktur mit den folgenden Schritten:
- Bereitstellen einer Grundstruktur,
- Anordnen mindestens eines ersten Teils eines Vlieses (3) auf und/oder in der Grundstruktur mittels Elektrospinnen,
- Einbringen und/oder Aufbringen eines Befestigungsmittels 20, ausgebildet als elastisches Element am proximalen und/oder am distalen Ende des Implantats derart, dass zumindest in einem expandierten Zustand des Implantats ein Abragen von Vliesabschnitten von der Grundstruktur und/oder die Bildung von größer ausgedehnten Bereichen, in denen Fasern oder Faserabschnitte von der Grundstruktur abragen, verringert ist,
- gegebenenfalls Anordnen eines zweiten Teils des Vlieses (3) auf und/oder in der Grundstruktur mittels Elektrospinnen,
- gegebenenfalls Befestigen des Implantats auf einem Katheter, vorzugsweise auf einem Ballon eines Katheters.

5. Verfahren nach Anspruch 4, **dadurch gekennzeichnet, dass** das Vlies (3) mittels Laser und/oder thermisch und/oder mit einem Lösungsmittel und/oder mit einem Lösungsmitteldampf zumindest in dem mindestens einen Abschnitt verdichtet wird.

6. Verfahren nach Anspruch 5, **dadurch gekennzeichnet, dass** das Vlies (3) derart verdichtet wird, dass ein elastisches Element (20), vorzugsweise ein elastischer Ring, durch die Verdichtung in dem mindestens einen Abschnitt erzeugt wird.

7. Verfahren nach einem der Ansprüche 4 bis 6, **dadurch gekennzeichnet, dass** an der Außenseite des Vlieses (3) ein Hydrogel und/oder ein hydrogelbasierter Klebstoff (16) angeordnet wird.

8. Verfahren nach einem der Ansprüche 4 bis 7, **dadurch gekennzeichnet, dass** in dem mindestens einen Abschnitt innen in das Implantat mindestens eine Torsionsfeder (12) eingesetzt und/oder auf die Außenseite mindestens eine Torsionsfeder aufgesetzt wird.

9. Verfahren nach einem der Ansprüche 4 bis 8, **dadurch gekennzeichnet, dass** das Vlies zumindest in dem mindestens einen Abschnitt mittels Laser perforiert wird.

## Claims

1. An implant formed as an intraluminal endoprosthesis with a hollow-cylindrical basic structure, wherein a nonwoven fabric (3) is disposed in and/or on the basic structure and contains biocompatible thermoplastic polyurethane and/or a polymer from the family of polylactic acid or polycaprolactone or copolymers thereof and/or a polymer from the family of polyphosphazenes, wherein in at least one portion of the implant, at the proximal and/or distal end of the implant, a fastening means (20) is provided such that, at least in an expanded state of the implant, a protrusion of nonwoven fabric portions from the basic structure and/or the formation of more extensive regions in which fibres or fibre portions protrude from the basic structure is reduced, **characterised in that** the fastening means is formed by an elastic element (20), preferably an elastic ring.

2. The implant according to the preceding claim, **characterised in that** the nonwoven fabric is produced by means of electrospinning.

3. The implant according to either one of the preceding claims, **characterised in that** the nonwoven fabric is treated in the at least one portion in such a way that it has a greater density.

4. A method for producing an implant formed as an intraluminal endoprosthesis having a preferably hollow-cylindrical basic structure, comprising the following steps:
- providing a basic structure;
- disposing at least a first part of a nonwoven fabric (3) on and/or in the basic structure, by way of electrospinning;
- introducing and/or applying a fastening means (20), formed as an elastic element, at proximal and/or distal ends of the implant, such that, at least in an expanded state of the implant, protrusion of nonwoven fabric portions from the basic structure and/or formation of more extensive regions in which fibres or fibre portions protrude from the basic structure are/is reduced;
- optionally disposing a second part of the nonwoven fabric (3) on and/or in the basic structure, by way of electrospinning;
- optionally fastening the implant on a catheter, preferably on a balloon of a catheter.

5. The method according to claim 4, **characterised in that** the nonwoven fabric (3) is compacted at least in the at least one portion by laser and/or thermally and/or with a solvent and/or with a solvent vapour.

6. The method according to claim 5, **characterised in that** the nonwoven fabric (3) is compacted in such a way that an elastic element (20), preferably an elastic ring, is produced by the compaction in the at least one portion.

7. The method according to any one of claims 4 to 6, **characterised in that** a hydrogel and/or a hydrogel-based adhesive (16) is disposed on the outside of the nonwoven fabric (3).

8. The method according to any one of claims 4 to 7, **characterised in that** at least one torsion spring (12) is inserted in the at least one portion into the inside of the implant and/or at least one torsion spring is placed on the outside.

9. The method according to any one of claims 4 to 8, **characterised in that** the nonwoven fabric is perforated by laser in the at least one portion.

## Revendications

1. Implant conçu sous forme d'endoprothèse intraluminale avec une structure de base en forme de cylindre creux, où un non tissé (3) est disposé dans et/ou sur la structure de base, lequel contient du polyuréthane thermoplastique biocompatible et/ou un polymère de la famille de l'acide poly lactique ou de la poly caprolactone ou de leurs copolymères, et/ou un polymère de la famille des polyphosphazènes, où un moyen de fixation 20 est prévu dans au moins un segment de l'implant à l'extrémité proximale et/ou distale de l'implant de telle manière qu'au moins dans un état expansé de l'implant, un dépassement de parties de non tissé à partir de la structure de base et/ou la formation de domaines davantage étirés dans lesquels des fibres ou des segments de fibres dépassent de la structure de base, est diminué, **caractérisé en ce que** le moyen de fixation est formé par un élément élastique (20), de préférence, un anneau élastique.

2. Implant selon l'une des revendications précédentes, **caractérisé en ce que** le non tissé est fabriqué par électrofilage.

3. Implant selon l'une des revendications précédentes, **caractérisé en ce que** le non tissé est traité dans au moins un segment de manière à ce qu'il présente une densité plus importante.

4. Procédé de fabrication d'un implant conçu sous forme d'endoprothèse intraluminale doté d'une structure de base en forme de cylindre creux avec les étapes suivantes :
- mise au point d'une structure de base,
- agencement d'au moins une première partie d'un non tissé (3 sur et/ou dans la structure de base au moyen de l'électrofilage,
- incorporation et/ou dépôt d'un produit de fixation 20, conçu sous forme d'élément élastique à l'extrémité proximale et/ou l'extrémité distale de l'implant, de telle manière qu'au moins dans un état expansé de l'implant, un dépassement de parties de non tissé à partir de la structure de base et/ou la formation de domaines davantage étirés davantage, dans lesquels des fibres ou des segments de fibres dépassent de la structure de base, est diminué,
- éventuellement, agencement d'une deuxième partie du non tissé (3) sur et/ou dans la structure de base au moyen de l'électrofilage,
- éventuellement, fixation de l'implant sur un cathéter, de préférence, sur un ballon de cathéter.

5. Procédé selon la revendication 4, **caractérisé en ce que** le non tissé (3) est rendu étanche dans l'au moins un segment avec un solvant et/ou avec une vapeur de solvant.

6. Procédé selon la revendication 5, **caractérisé en ce que** le non tissé (3) est rendu étanche de telle manière qu'un élément élastique (20) ; de préférence un anneau élastique, est généré par l'étanchéification dans l'au moins un segment.

7. Procédé selon l'une des revendications 4 à 6, **caractérisé en ce qu'**un hydrogel et/ou un adhésif à base d'hydrogel (16) est disposé sur la face extérieure du non tissé (3).

8. Procédé selon l'une des revendications 4 à 7, **caractérisé en ce que**, dans l'au moins un segment, un ressort de torsion (12) est implanté à l'intérieur de l'implant et/ou un ressort de torsion est appliqué sur la face extérieure.

9. Procédé selon l'une des revendications 4 à 8, **caractérisé en ce que** le non tissé est perforé au moins dans l'au moins un segment au moyen d'un laser.
